(19) Europäisches Patentamt
European Patent Office
Office européen des brevets

(11) Publication number: **0 528 583 A1**

# (12) EUROPEAN PATENT APPLICATION

(21) Application number: **92307133.6**

(22) Date of filing: **05.08.92**

(51) Int. Cl.[5]: **C07K 7/54**, C07K 3/28, A61K 37/02

(30) Priority: **08.08.91 US 742608**

(43) Date of publication of application:
**24.02.93 Bulletin 93/08**

(84) Designated Contracting States:
**AT BE CH DE DK ES FR GB GR IT LI LU MC NL PT SE**

(71) Applicant: **ARIZONA BOARD OF REGENTS, ARIZONA STATE UNIVERSITY**

**Tempe, Arizona 85287(US)**

(72) Inventor: **Pettit, George R.**
**6232 Bret Hills Drive**
**Paradise Valley, Arizona 85253(US)**
Inventor: **Herald, Cherry L.**
**1324 West 7th Street**
**Tempe, Arizona 85281(US)**

(74) Representative: **Coxon, Philip et al**
**Eric Potter & Clarkson St. Mary's Court St. Mary's Gate**
**Nottingham NG1 1LE (GB)**

## (54) Isolation and structure of the cell growth inhibitory cycloheptapeptide axinastatin 1.

(57) An unusual cytostatic (PS $ED_{50}$ 0.21 μg/ml) cycloheptapeptide has been isolated from the Western Pacific marine sponge Axinella sp. and is herein designated axinastatin 1. Separation of a methylene chloride methanol extract of the sponge by means of PS P388 guided bioassay and a series of detailed solvent partition, gel permeation and adsorption column chromatographic techniques separated the cycloheptapeptide having the structure.

EP 0 528 583 A1

The present invention relates to the isolation and structural elucidation of a new cyclic peptide, herein denominated "axinastatin 1", which is obtained from the Western Pacific marine sponge Axinella sp. The structure of this cyclic peptide was determined by combined $^1H$, $^1H$ COSY, $^1H$, $^{13}C$ COSY, $^1H$, $^1H$ relayed COSY, HMBC and NOESY experiments. The substance demonstrated cytostatic (PS $ED_{50}$ 0.21 µg/ml) and in vivo (20% life extension) antineoplastic (P 388: lymphocytic leukemia) properties and has the general structural formula:

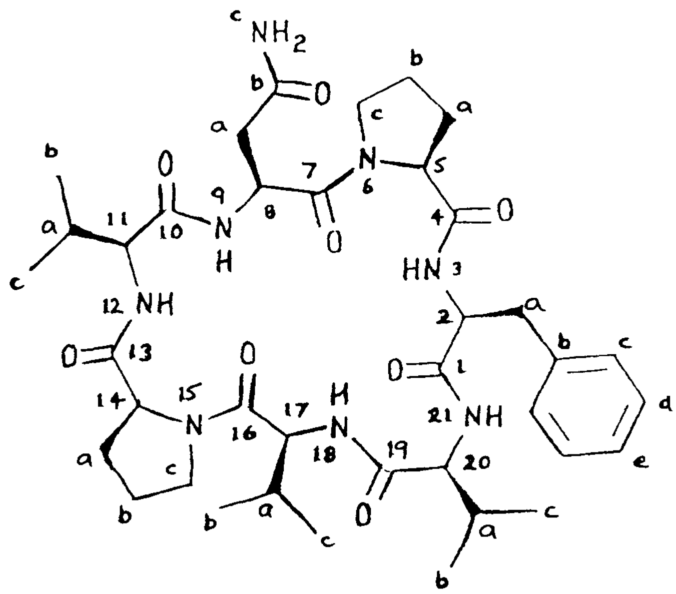

Only a small number of antineoplastic or peptide constituents have been recovered from marine invertebrate Porifera. The isolation and structural determination of the P388 lymphocytic leukemia (PS system) cell growth inhibitory cyclo-octapeptide hymenistatin 1 from a Palau sponge in the genus Hymeniacidon represented the first such combination of source, structural type and biological activity, all performed at or under the direction of the Cancer Research Institute at Arizona State University, Tempe, Arizona. Also found was an Axinella sp. (Demospongiae class) collected (in 1979) in Palau (at -40 m) which yielded a methylene chloride-2-propanol extract that provided a 101% increase in life span (at 100 mg/kg) against the PS leukemia with $ED_{50}$ 2.5 µg/mL in the corresponding NCI murine P388 lymphocytic leukemia cell line.

The present invention relates to the isolation and structural elucidation of a new cyclo- heptapeptide denominated "axinastatin 1", which is obtained from the Western Pacific marine sponge Axinella sp. The substance, when measured by the generally accepted protocol for P388 murine lymphatic leukemia demonstrated as PS $ED_{50}$ of 0.21 µg/ml and 20% life extension in the P388 lymphocytic leukemia system.

Accordingly, a principal object of the present invention is to isolate a new substance which is useful in the treatment and management of those neoplastic diseases which are characterized by uncontrolled cell growth and have an established correlation to the NCI protocol for P388 murine lymphocytic leukemia.

An aspect of the present invention is to elucidate the structure of a newly discovered cycloheptapeptide denominated "axinastatin 1" so as to provide a readily discernible target for the direction of future synthetic endeavors.

A further aspect of the present invention is to provide means and methods of creating useful pharmaceutical preparations for the treatment and management of those neoplastic diseases which are correlatable to NCI's P388 murine lymphatic leukemia cell line and life extension protocols which preparations contain as their essential active ingredient a unique cytostatic factor obtained from Western Pacific marine sponge Axinella sp.

These and still further objects as shall hereinafter appear are readily fulfilled by the present invention in a remarkably unexpected manner as will be readily discerned from the following detailed description of an exemplary embodiment thereof.

Axinella sp. was recollected in 2-propanol and a 220Kg (wet weight) was extracted with methylene chloride-methanol by means of PS guided bioassay and a series of detailed solvent partition, gel permeation (and gel partition, SEPHADEX LH-20), partition (silica gel including reversed phase) and absorption column chromatographic techniques, as more fully described below.

Solvents used for chromatographic procedures were redistilled. The SEPHADEX LH-20 (25-100 μm) employed for gel permeation and partition chromatography was obtained from Pharmacia Fine Chemicals AB, Uppsala, Sweden. GILSON FC-220 race track and F-80 microfractionators connected to GILSON HM UV-VIS HOLACHROME detectors were used for chromatographic fractionation experiments. Column chromatographic procedures with silica gel utilized the silica gel 60 prepacked "LOBAR" columns supplied by E. Merck, Darmstadt, West Germany. The silica gel GF Uniplates for TLC were from Analtech Inc., Newark, Delaware. All TLC plates were viewed with UV light and (or) developed with a ceric sulphate - sulfuric acid spray (heating to approximately 150°C for 10 min).

The uncorrected melting points were observed using a KOFLER-type melting point apparatus. The UV spectrum was recorded using a HEWLETT-PACKARD 8450A UV-VIS spectrophotometer equipped with a HP7225A plotter. Optical rotation and IR spectral data were obtained using a PERKIN-ELMER 241 polarimeter and a NICOLET MX-1 FTIR spectro- photometer, respectively. Mass spectra (70 eV and FAB) were recorded employing a KRATOS MS-50 spectrometer. The NMR experiments were conducted with a BRUKER WH-400 instrument and deuteriochloroform as solvent (TMS internal standard).

A new PS inhibitory ($ED_{50}$0.21 μg/ml and 20% life extension in the in vivo model) peptide (100 mg, 4.54 X $10^{-5}$% yield) antineoplastic constituent has been isolated which is herein designated as "axinastatin 1".

The significance of the NCI screens and their relationship to ultimate human therapy is well-known in the art and need not be repeated here. (See: Boyd, Status of the NCI preclinical antitumor drug discovery screen: implications for selection of new agents for clinical trial. In: DeVita et al, Principles and Practices of Oncology, Update Series, Vol. 3, No. 10, Lippincott, Philadelphia, 1989, pp 1-12; and Boyd et al, Data display and analysis strategies from NCI disease oriented in vitro antitumor drug screen. In: Valeriote et al, Antitumor Drug Discovery and Development, Kluwer Academic Press, Amsterdam, 1990).

The isolation and purification methods chosen can be monitored at each step by performing in-vitro and/or in-vivo antitumor tests as described by R. I. Geran, N.H. Greenberg, M. M. MacDonald, A.M. Schumacher and B. S. Abbot in Cancer Chemotherapy Rep., Part 3, Vol 3: 1-103 (1972); and by Schmidt, J. M.; Pettit, G. R. in Experientia 1978, 34: 659-660. Such tests include the determination of the concentration of active material required to inhibit the growth of tumor cells in culture, (e.g. the concentration required to inhibit growth by 50 percent or the $E.D._{50}$) and of the dose of active material required to prolong the life of mice bearing transplanted tumors ("life extension").

Axinastatin 1 crystallized from methylene chloride: mp 283-7° (dec), $[\alpha]_D^{25}$ - 161.6° (C, 0.099, $CH_3OH$), TLC (Rf 0.18 in 95:5 $CH_2Cl_2$:MeOH); UV ($CH_3OH$) $\nu_{max}$ 208 nm (ε18,000); IR (NaCl plate), $\nu_{max}$ 3320, 3960, 1640, 1520, 1465, 1430 $cm^{-1}$; high resolution FAB MS 753.4293 $[M+H]^+$, theoretical mass for $[M+H]^+$ of $C_{38}H_{56}N_8O_8$ requires 753.4299; amino acid analyses Asp, (or Asn), Phe, Pro and Val in the ratio 1:1:2:3. The molecular formula for axinastatin 1 (as shown below) was deduced from high field (400 MHz) $^1H$- and $^{13}C$-NMR studies in conjunction with the high resolution FAB MS peak matching experiments just noted. Combined $^1H$, $^1H$ COSY, $^1H$, $^{13}C$ COSY, $^1H$, $^1H$ relayed COSY, HMBC and NOESY experiments confirmed the amino acid sequence and cyclic structure. The amino acid components and sequence of axinastatin 1 were confirmed as cyclo (Asn-Pro-Phe-Val-Val-Pro-Val) by tandem (MS/MS) mass spectrometry.

## Table I

Axinastatin 1 (3) $^{1}H$ and $^{13}C$-NMR Assignments and Selected NOE's and HMBC Correlations in Deuteriochloroform (0.11 M) Solution

| Structure Assignment | | $^{13}C$ | $^{1}H$ (Mult, J(Hz), intgrtn) | NOESY | HMBC (H to C. no.) |
|---|---|---|---|---|---|
| Phe | 1 | 172.4 | | | |
| | 2 | 55.6 | 4.68 dt, 4.8, 10.8 | 2a | 1, 4 |
| | 2a | 37.6 | 2.94 | | |
| | | | 3.20 | | |
| | 2b | 137.6 | | | |
| | 2c | 128.9 | 7.18, 2H | | |
| | 2d | 128.2 | 7.23, 2H | | |
| | 2a | 126.5 | 7.17 | | |
| | 3 | | 7.74 | 2, 2a, 5 | 4 |
| $Pro_1$ | 4 | 170.7 | | | |
| | 5 | 63.3 | 4.06 dd, 9.7, 7.6 | 5a | 4 |
| | 5a | 29.5 | 1.34 | | |
| | | | 2.23 | | |
| | 5b | 25.8 | 1.80 | | |
| | | | 1.92 | | |
| | 5a | 48.0 | 3.46 | | |
| | | | 3.57 t, 8.3 | | |
| Asn | 7 | 169.3 | | | |
| | 8 | 50.3 | 4.57 q, 4.2 | 6c, 8a | |
| | 8a | 35.8 | 2.95 | | 8b |
| | | | 3.23 | | 7, 8b |
| | 8b | 172.8 | | | |
| | 8c | | 5.52 | | 8b |
| | | | 7.69 | 8a | |
| | 9 | | 8.06 d, 4.9 | 8, 11, 11a | 7 10 |
| $Val_1$ | 10 | 172.2 | | | |
| | 11 | 62.2 | 4.02 t, 8.2 | 11a | 10 |
| | 11a | 29.5 | 2.35 | | |
| | 11b | 18.7 | 1.01, ,3H | | |
| | 11c | 19.9 | 1.05, ,3H | | |
| | 12 | | 8.25 d, 7.9 | 11, 11a, 14 | 11, 13 |
| $Pro_2$ | 13 | 171.4 | | | |
| | 14 | 61.2 | 4.53 d, 7 | 14a, 17, 17a | 13 |
| | 14a | 31.1 | 1.88 | | |
| | | | 2.55 | | |
| | 14b | 21.7 | 1.67 | | |
| | | | 1.92 | | |
| | 14c | 46.0 | 3.46 | | |
| | | | 3.69 | | |
| $Val_2$ | 16 | 171.4 | | | |
| | 17 | 58.6 | 4.13 dd, 7.4, 4.1 | 14, 17a | 16 |
| | 17a | 30.0 | 1.96 | | |
| | 17b | 18.9 | 1.02, ,3H | | |
| | 17c | 19.2 | 0.93, ,3H | | |
| | 18 | | 7.44 | | 16 |
| $Val_3$ | 19 | 171.7 | | | |
| | 20 | 57.3 | 4.23 t, 9.5 | 20a | 1, 19 |
| | 20a | 29.2 | 2.02 | | |
| | 20b | 18.5 | 0.94, ,3H | | |
| | 20c | 19.5 | 0.92, ,3H | | |
| | 21 | | 7.44 | | 1 |

Protonation upon FAB results in ring opening of the cyclic peptide at an N-acyl bond to give a linear acylium ion. The major fragmentation processes observed by tandem mass spectrometry involve losses of amino acid residues from the C terminus. Protonation is favored at proline, and with axinastatin 1 there are two possibilities. The FAB MS/MS spectrum of the [M+H] species contain two series (A and B) of ions resulting from protonation at the two proline units.

All of the ions in both series were observed. Additional supporting information for the sequence was obtained by MS/MS experiments on source-produced fragment ions to confirm the inter- relationship of the fragment ion and by exact mass measurements on the fragment ions to verify elemental composition and correct assignment.

The absolute configuration of axinastatin 1 was ascertained by analyzing the acid hydrolysate N-pentafluoropropionyl-isopropyl ester derivatives using chiral GC (Chirasil-Val III column). Each amino acid

was found to have the L-configuration. The disproportionately high representation of L-Pro and L-Val in axinastatin 1 and other strongly antineoplastic peptides discovered in marine animals suggests that the presence of these amino acids may be an important structural requirement for controlling cell growth in peptide mediated systems.

The administration of axinastatin 1 is useful for treating animals or humans bearing a neoplastic disease, for example, acute myelocytic leukemia, acute lymphocytic leukemia, malignant melanoma, adenocarcinoma of lung, neuroblastoma, small cell carcinoma of lung, breast carcinoma, colon carcinoma, ovarian carcinoma, bladder carcinoma, and the like.

The dosage administered will be dependent upon the identity of the neoplastic disease; the type of host involved, including its age, health and weight; the kind of concurrent treatment, if any; the frequency of treatment and therapeutic ratio.

Illustratively, dosage levels of the administered active ingredients are: intravenous, 0.1 to about 200 mg/kg; intra- muscular, 1 to about 500 mg/kg; intraperitoneal, 1 to about 500 mg/kg; subcutaneous, 1 to about 500 mg/kg; orally, 5 to about 1000 mg/kg; intranasal instillation, 5 to about 1000 mg/kg; and aerosol, 5 to about 100 mg/kg of host body weight.

Expressed in terms of concentration, an active ingredient can be present in the compositions of the present invention for localized use about the cutis, intranasally, pharyngolaryngeally, bronchially, broncholially, intravaginally, rectally, or ocularly in a concentration of from about 0.01 to about 50% w/w of the composition; preferably about 1 to about 20% w/w of the composition; and for parenteral use in a concentration of from about 0.05 to about 50% w/v of the composition and preferably from about 5 to about 20% w/v.

The compositions of the present invention are preferably presented for administration to humans and animals in unit dosage forms, such as tablets, capsules, pills, powders, granules, suppositories, sterile parenteral solutions or suspensions, sterile non-parenteral solutions or suspensions, and oral solutions or suspensions and the like, containing suitable quantities of an active ingredient.

For oral administration either solid or fluid unit dosage forms can be prepared.

Powders are prepared quite simply by comminuting the active ingredient to a suitably fine size and mixing with a similarly comminuted diluent. The diluent can be an edible carbohydrate material such as lactose or starch. Advantageously, a sweetening agent or sugar is present as well as a flavoring oil.

Capsules are produced by preparing a powder mixture as hereinbefore described and filling into formed gelatin sheaths. Advantageously, as an adjuvant to the filling operation, a lubricant such as a talc, magnesium stearate, calcium stearate and the like is added to the powder mixture before the filling operation.

Soft gelatin capsules are prepared by machine encapsulation of a slurry of active ingredients with an acceptable vegetable oil, light liquid petrolatum or other inert oil or triglyceride.

Tablets are made by preparing a powder mixture, granulating or slugging, adding a lubricant and pressing into tablets. The powder mixture is prepared by mixing an active ingredient, suitably comminuted, with a diluent or base such as starch, lactose, kaolin, dicalcium phosphate and the like. The powder mixture can be granulated by wetting with a binder such as corn syrup, gelatin solution, methylcellulose solution or acacia mucilage and forcing through a screen. As an alternative to granulating, the powder mixture can be slugged, i.e., run through the tablet machine and the resulting imperfectly formed tablets broken into pieces (slugs). The slugs can be lubricated to prevent sticking to the tablet-forming dies by means of the addition of stearic acid, a stearic salt, talc or mineral oil. The lubricated mixture is then compressed into tablets.

Advantageously the tablet can be provided with a protective coating consisting of a sealing coat or enteric coat of shellac, a coating of sugar and methylcellulose and polish coating of carnauba wax.

Fluid unit dosage forms for oral administration such as syrups, elixirs and suspensions can be prepared wherein each teaspoonful of composition contains a predetermined amount of active ingredient for administration. The water-soluble forms can be dissolved in a aqueous vehicle together with sugar, flavoring agents and preservatives to form a syrup. An elixir is prepared by using a hydroalcoholic vehicle with suitable sweeteners together with a flavoring agent. Suspensions can be prepared of the insoluble forms with a suitable vehicle with the aid of a suspending agent such as acacia, tragacanth, methylcellulose and the like.

For parenteral administration, fluid unit dosage forms are prepared utilizing an active ingredient and a sterile vehicle, water being preferred. The active ingredient, depending on the form and concentration used, can be either suspended or dissolved in the vehicle. In preparing solutions, the water-soluble active ingredient can be dissolved in water for injection and filter sterilized before filling into a suitable vial or ampule and sealing. Advantageously, adjuvants such as a local anesthetic, preservative and buffering agents can be dissolved in the vehicle. Parenteral suspensions are prepared in substantially the same

manner except that an active ingredient is suspended in the vehicle instead of being dissolved and sterilization cannot be accomplished by filtration. The active ingredient can be sterilized by exposure to ethylene oxide before suspending in the sterile vehicle. Advantageously, a surfactant or wetting agent is included in the composition to facilitate uniform distribution of the active ingredient.

In addition to oral and parenteral adminis- tration, the rectal and vaginal routes can be utilized. An active ingredient can be administered by means of a suppository. A vehicle which has a melting point at about body temperature or one that is readily soluble can be utilized. For example, cocoa butter and various polyethylene glycols (Carbowaxes) can serve as the vehicle.

For intranasal instillation, a fluid unit dosage form is prepared utilizing an active ingredient and a suitable pharmaceutical vehicle, preferably P.F. water, a dry powder can be formulated when insufflation is the administration of choice.

For use as aerosols, the active ingredients can be packaged in a pressurized aerosol container together with a gaseous or liquefied propellant, for example, dichlorodifluoromethane, carbon dioxide, nitrogen, propane, and the like, with the usual adjuvants such as cosolvents and wetting agents, as may be necessary or desirable.

The term "unit dosage form" as used in the specification and claims refers to physically discrete units suitable as unitary dosages for human and animal subjects, each unit containing a predetermined quantity of active material calculated to produce the desired therapeutic effect in association with the required pharmaceutical diluent, carrier or vehicle. The specifications for the novel unit dosage forms of this invention are dictated by and are directly dependent on (a) the unique characteristics of the active material and the particular therapeutic effects to be achieved, and (b) the limitation inherent in the art of compounding such an active material for therapeutic use in humans, as disclosed in this specification, these being features of the present invention. Examples of suitable unit dosage forms in accord with this invention are tablets, capsules, troches, suppositories, powder packets, dropperfuls, ampules, vials, segregated multiples of any of the foregoing, and other forms as herein described.

The active ingredient to be employed as antineoplastic agents can be easily prepared in such unit dosage form with the employment of pharmaceutical materials which themselves are available in the art and can be prepared by established procedures.

To further aid in the understanding of the present invention, and not as a limitation thereof, the following examples are presented.

EXAMPLE 1

Several dosage forms were prepared embodying the present invention. They are shown in the following examples which the notation "active ingredient" signifies Axinastatin 1.

COMPOSITION "A"

Hard-Gelatin Capsules

One thousand two-piece hard gelatin capsules for oral use, each capsule containing 200 mg of an active ingredient are prepared from the following types and amounts of ingredients:

| | |
|---|---|
| Active ingredient, micronized | 200 gm |
| Corn Starch | 20 gm |
| Talc | 20 gm |
| Magnesium stearate | 2 gm |

The active ingredient, finely divided by means of an air micronizer, is added to the other finely powdered ingredients, mixed thoroughly and then encapsulated in the usual manner.

The foregoing capsules are useful for treating a neoplastic disease by the oral administration of one or two capsules one for four times a day.

Using the procedure above, capsules are similarly prepared containing a active ingredient in 50, 250 and 500 mg amounts by substituting 50 gm, 250 gm and 500 gm of a active ingredient for the 200 gm used above.

COMPOSITION "B"

Soft-Gelatin Capsules

One-piece soft gelatin capsules for oral use, each containing 200 mg of a active ingredient (finely divided by means of an air micronizer), are prepared by first suspending the compound in 0.5 ml of corn oil to render the material capsulatable and then capsulating in the above manner.

The foregoing capsules are useful for treating a neoplastic disease by the oral administration of one or two capsules one to four times a day.

COMPOSITION "C"

Tablets

One thousand tablets, each containing 200 mg of a active ingredient are prepared from the following types and amounts of ingredients:

| | |
|---|---|
| Active ingredient, micronized | 200 gm |
| Lactose | 300 gm |
| Corn starch | 50 gm |
| Magnesium stearate | 4 gm |
| Light liquid petrolatum | 5 gm |

The active ingredient finely divided by means of an air micronizer, is added to the other ingredients and then thoroughly mixed and slugged. The slugs are broken down by forcing through a Number Sixteen screen. The resulting granules are then compressed into tablets, each tablet containing 200 mg of the active ingredient.

The foregoing tablets are useful for treating a neoplastic disease by the oral administration of one or two tablets one to four times a day.

Using the procedure above, tablets are similarly prepared containing an active ingredient in 250 mg and 100 mg amounts by substituting 250 gm and 100 gm of an active ingredient for the 200 gm used above.

COMPOSITION "D"

Oral Suspension

One thousand ml of an aqueous suspension for oral use, containing in each teaspoonful (5 ml) dose, 50 mg of a active ingredient, is prepared from the following types and amounts of ingredients:

| | |
|---|---|
| Benzoic acid | 1 gm |
| Sucrose | 790 gm |
| Tragacanth | 5 gm |
| Lemon Oil | 2 gm |
| Deionized water, q.s. 1000 ml. | |

The citric acid, benzoic acid, sucrose, tragacanth and lemon oil are dispersed in sufficient water to make 850 ml of suspension. The active ingredient finely divided by means of an air micronizer, is stirred into the syrup until uniformly distributed. Sufficient water is added to make 1000 ml.

The composition so prepared is useful for treating a neoplastic disease at a dose of 1 tablespoonful (15 ml) three times a day.

COMPOSITION "E"

Parenteral Product

A sterile aqueous suspension for parenteral injection, containing in 1 ml 300 mg of a active ingredient for treating a neoplastic disease, is prepared from the following types and amounts of ingredients:

| | |
|---|---|
| Active ingredient, micronized | 30 gm |
| Polysorbate 80 | 5 gm |
| Methylparaben | 2.5 gm |
| Propylparaben | 0.17 gm |
| Water for injection, q.s. 1000 ml. | |

All the ingredients, except the active ingredient, are dissolved in the water and the solution sterilized by filtration. To the sterile solution is added the sterilized active ingredient, finely divided by means of an air micronizer, and the final suspension is filled into sterile vials and the vials sealed.

The composition so prepared is useful for treating a neoplastic disease at a dose of 1 milliliter (1M) three times a day.

COMPOSITION "F"

Suppository, Rectal and Vaginal

One thousand suppositories, each weighing 2.5 gm and containing 200 mg of a active ingredient are prepared from the following types and amounts of ingredients:

| | |
|---|---|
| Active ingredient, micronized | 15 gm |
| Propylene glycol | 150 gm |
| Polyethylene glycol #4000, g.s. | 2,500 mg |

The active ingredient is finely divided by means of an air micronizer and added to the propylene glycol and the mixture passed through a colloid mill until uniformly dispersed. The polyethylene glycol is melted and the propylene glycol dispersion added slowly with stirring. The suspension is poured into unchilled molds at 40o C. The composition is allowed to cool and solidify and then removed from the mold and each suppository foil wrapped.

The foregoing suppositories are inserted rectally or vaginally for treating a neoplastic disease.

COMPOSITION "G"

Intranasal Suspension

One thousand ml of a sterile aqueous suspension for intranasal instillation, containing in each ml 200 mg of a active ingredient, is prepared from the following types and amounts of ingredients:

| | |
|---|---|
| Active ingredient, micronized | 15 gm |
| Polysorbate 80 | 5 gm |
| Methylparaben | 2.5 gm |
| Propylparaben | 0.17 gm |
| Deionized water, q.s. 1000 ml. | |

All the ingredients, except the active ingredient, are dissolved in the water and the solution sterilized by filtration. To the sterile solution is added the sterilized active ingredient, finely divided by means of an air micronizer, and the final suspension is aseptically filled into sterile containers.

The composition so prepared is useful for treating a neoplastic disease, by intranasal instillation of 0.2 to 0.5 ml given one to four times per day.

An active ingredient can also be present in the undiluted pure form for use locally about the cutis, intranasally, pharyngolaryngeally, bronchially, broncholially, or orally.

COMPOSITION "H"

Powder

Five grams of a active ingredient in bulk form is finely divided by means of an air micronizer. The micronized powder is placed in a shaker-type container.

The foregoing composition is useful for treating a neoplastic disease, at localized sites by applying a powder one to four times per day.

COMPOSITION "I"

Oral Powder

One hundred grams of a active ingredient in bulk form is finely divided by means of an air micronizer. The micronized powder is divided into individual doses of 200 mg and packaged.

The foregoing powders are useful for treating a neoplastic disease, by the oral administration of one or two powders suspended in a glass of water, one to four times per day.

COMPOSITION "J"

Insufflation

One hundred grams of a active ingredient in bulk form is finely divided by means of an air micronizer.

The foregoing composition is useful for treating a neoplastic disease, by the inhalation of 300 mg one to four times per day.

COMPOSITION "K"

Hard-Gelatin Capsules

One hundred two-piece hard gelatin capsules were prepared for oral use, each capsule containing 200 mg of an active ingredient. The active ingredient is finely divided by means of an air micronizer and encapsulated in the usual manner. The foregoing capsules are useful for treating a neoplastic disease, by the oral administration of one or two capsules, one to four times a day.

Using the procedure above, capsules are similarly prepared containing active ingredient in 50, 250 and 500 gm of the active ingredient for the 200 gm used above.

From the foregoing, it is readily apparent that a useful embodiment of the present invention has been herein described and illustrated which fulfills all of the aforestated objectives in a remarkably unexpected fashion. It is of course understood that such modifications, alterations and adaptations as may readily occur to the artisan confronted with this disclosure are intended within the spirit of this disclosure which is limited only by the scope of the claims appended hereto.

## Claims

**1.** A compound having the structural formula:

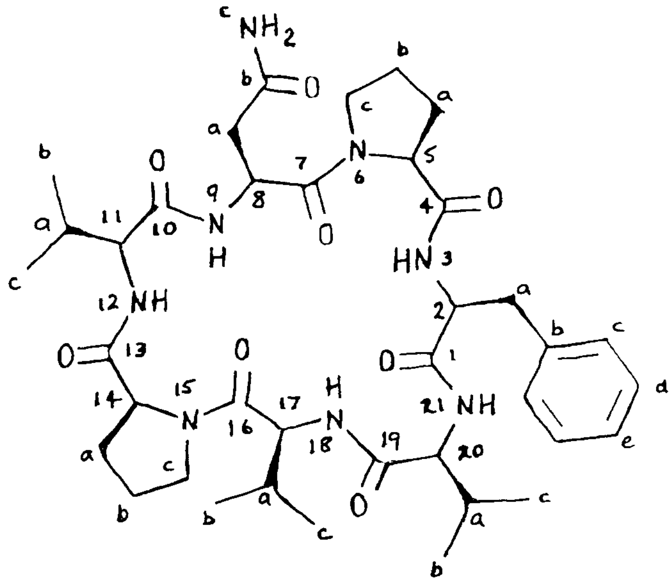

2. A compound which, when dissolved in a (o.11 M) deuteriochloroform solution has the $^{1}H$, $^{13}C$ NMR assignment and NOE's and HMBC correlation as shown:

Axinastatin 1 (3) $^1H$ and $^{13}C$-NMR Assignments and Selected NOE's and HMBC Correlations in Deuteriochloroform (0.11 M) Solution

| Structure Assignment | | $^{13}C$ | $^1H$ (Mult, J(Hz), intgrtn) | NOESY | HMBC (H to C. no.) |
|---|---|---|---|---|---|
| Phe | 1 | 172.4 | | | |
| | 2 | 55.6 | 4.68 dt, 4.8, 10.8 | 2a | 1, 4 |
| | 2a | 37.6 | 2.94 | | |
| | | | 3.20 | | |
| | 2b | 137.6 | | | |
| | 2c | 128.9 | 7.18, 2H | | |
| | 2d | 128.2 | 7.23, 2H | | |
| | 2a | 126.5 | 7.17 | | |
| | 3 | | 7.74 | 2, 2a, 5 | 4 |
| $Pro_1$ | 4 | 170.7 | | | |
| | 5 | 63.3 | 4.06 dd, 9.7, 7.6 | 5a | 4 |
| | 5a | 29.5 | 1.34 | | |
| | | | 2.23 | | |
| | 5b | 25.8 | 1.80 | | |
| | | | 1.92 | | |
| | 5a | 48.0 | 3.46 | | |
| | | | 3.57 t, 8.3 | | |
| Asn | 7 | 169.3 | | | |
| | 8 | 50.3 | 4.57 q, 4.2 | 6c, 8a | |
| | 8a | 35.8 | 2.95 | | 8b |
| | | | 3.23 | | 7, 8b |
| | 8b | 172.8 | | | |
| | 8c | | 5.52 | | 8b |
| | | | 7.69 | 8a | |
| | 9 | | 8.06 d, 4.9 | 8, 11, 11a | 7 10 |
| $Val_1$ | 10 | 172.2 | | | |
| | 11 | 62.2 | 4.02 t, 8.2 | 11a | 10 |
| | 11a | 29.5 | 2.35 | | |
| | 11b | 18.7 | 1.01, ,3H | | |
| | 11c | 19.9 | 1.05, ,3H | | |
| | 12 | | 8.25 d, 7.9 | 11, 11a, 14 | 11, 13 |
| $Pro_2$ | 13 | 171.4 | | | |
| | 14 | 61.2 | 4.53 d, 7 | 14a, 17, 17a | 13 |
| | 14a | 31.1 | 1.88 | | |
| | | | 2.55 | | |
| | 14b | 21.7 | 1.67 | | |
| | | | 1.92 | | |
| | 14c | 46.0 | 3.46 | | |
| | | | 3.69 | | |
| $Val_2$ | 16 | 171.4 | | | |
| | 17 | 58.6 | 4.13 dd, 7.4, 4.1 | 14, 17a | 16 |
| | 17a | 30.0 | 1.96 | | |
| | 17b | 18.9 | 1.02, ,3H | | |
| | 17c | 19.2 | 0.93, ,3H | | |
| | 18 | | 7.44 | | 16 |
| $Val_3$ | 19 | 171.7 | | | |
| | 20 | 57.3 | 4.23 t, 9.5 | 20a | 1, 19 |
| | 20a | 29.2 | 2.02 | | |
| | 20b | 18.5 | 0.94, ,3H | | |
| | 20c | 19.5 | 0.92, ,3H | | |
| | 21 | | 7.44 | | 1 |

**3.** A method of inhibiting cell growth in NCI's P388 murine lymphatic leukemia comprising treating said leukemia with a cell growth inhibiting amount of a compound having the structural formula:

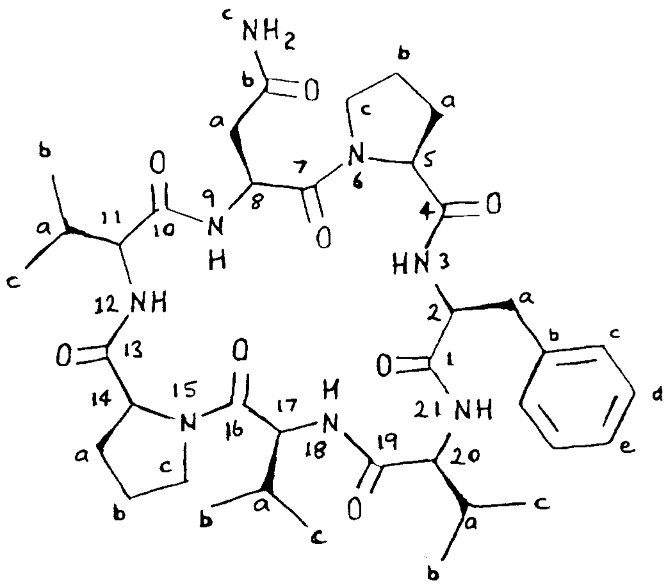

4. A compound having the structural formula:

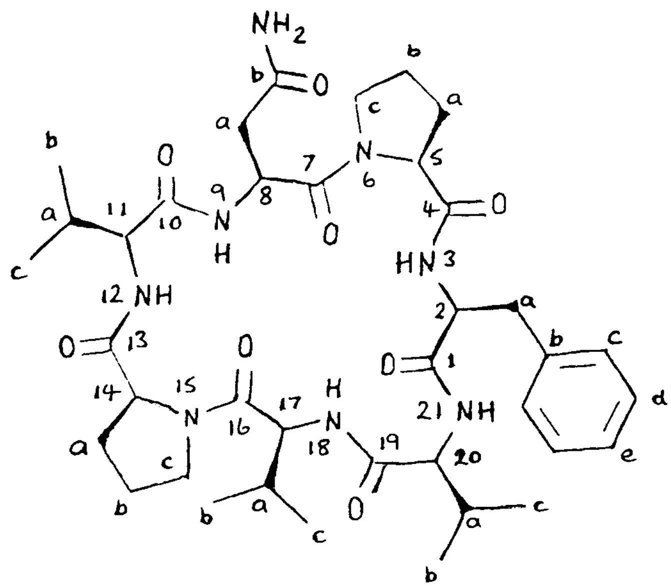

for use in medicine.

5. The use of a compound having the structural formula:

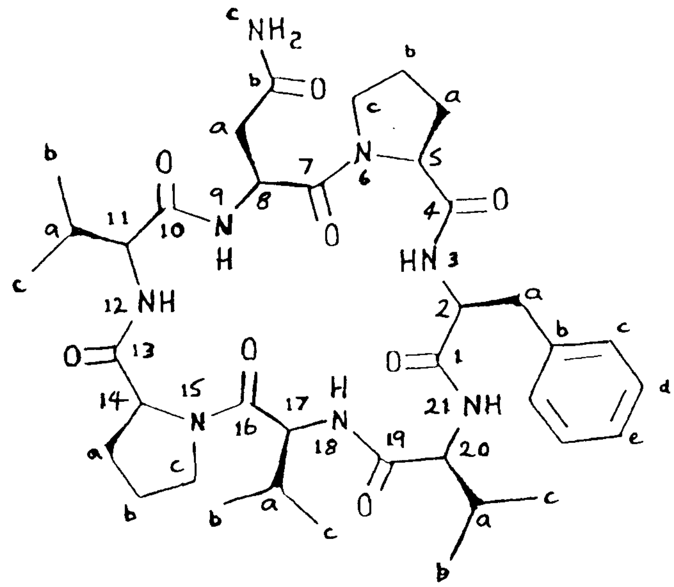

in the manufacture of a medicament for inhibiting cell growth in a host afflicted with a neoplastic disease is correlatable to P388 marine lymphatic leukemia.

European Patent Office

# EUROPEAN SEARCH REPORT

Application Number EP 92307133.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US - A - 4 370 484 (D. JOHN FAULKNER) * Claims 1-5 * | 1,3-5 | C 07 K 7/54<br>C 07 K 3/28<br>A 61 K 37/02 |
| A | CHEMICAL ABSTRACTS, vol. 114, no. 11, March 18, 1991 Columbus, Ohio, USA PETTIT, GEORGE R. et al. "Antineoplastic agents. 168. Isolation and structure of axinohydantoin." page 427, column 2, abstract--no. 98 594s & Can. J. Chem. 1990, 68(9), 1621-4 | 1-5 | |
| A | CHEMICAL ABSTRACTS, vol. 102, no. 11, March 18, 1985 Columbus, Ohio, USA BAKUS, G. J. et al. "Toxins from marine organisms: studies on antifouling." page 166, column 2, abstract--no. 91 141k & Toxins, Drugs, Pollut. Mar. Anim. 1984, 43-6 | 1-5 | **TECHNICAL FIELDS SEARCHED (Int. Cl.5)**<br>C 07 K 3/00<br>C 07 K 7/00<br>C 07 K 11/00<br>C 07 K 15/00<br>C 12 P 21/00<br>A 61 K 37/00<br>C 07 D 403/00 |
| A | CHEMICAL ABSTRACTS, vol. 94, no. 11, March 16, 1981 Columbus, Ohio, USA WRATTEN, S. J. et al. "Antimicrobial metabolites of the marine sponge Axinella polycapella." page 407, column 1, abstract-no. 80497w & Experientia 1981, 37(1), 13-14 | 1-5 | |

The present search report has been drawn up for all claims

| Place of search | Date of completion of the search | Examiner |
|---|---|---|
| VIENNA | 12-11-1992 | SCHARF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)